# FASCICULE DE BREVET EUROPEEN

(11) **EP 2 255 197 B1**
(45) Date de publication et mention de la délivrance du brevet: **02.11.2011**
(21) Numéro de dépôt: 09720139.6
(22) Date de dépôt: 30.01.2009
(51) Int. Cl.: G01N 33/569, G01N 33/68

(54) **PROCEDE DE DETECTION ET/OU DE QUANTIFICATION ET/OU D'IDENTIFICATION IN VITRO DE BACTERIES DANS UN MATERIAU BIOLOGIQUE**
VERFAHREN FÜR IN VITRO NACHWEIS UND/ODER QUANTIFIZIERUNG UND/ODER IDENTIFIZIERUNG VON BAKTERIEN IN BIOLOGISCHEM MATERIAL
METHOD FOR IN VITRO DETECTION AND/OR QUANTIFICATION AND/OR IDENTIFICATION OF BACTERIA IN A BIOLOGICAL MATERIAL

(30) Priorité: 01.02.2008 FR 0800552
(43) Date de publication de la demande: 01.12.2010
(73) Titulaire: Apoh Technologies SA, 34280 La Grande Motte (FR)
(72) Inventeur: STEFAS, Ilias, F-34280 La Grande Motte (FR)
(74) Mandataire: Galup, Cédric Olivier Nicolas
(86) Numéro de dépôt international: PCT/FR2009/000105
(87) Numéro de publication internationale: WO 2009/112702

(56) Documents cités:
- EP-A- 0 775 315
- WO-A-94/18569
- FR-A- 2 723 203
- FR-A- 2 723 204

## Description

La présente invention concerne un procédé de détection et/ou de quantification et/ou d'identification in vitro de bactéries dans un matériau biologique.

Dans la présente description, on entend par "matériau biologique" un tissu biologique, une préparation ou un extrait issu de tissu biologique, liquide ou solide, ou un milieu, naturel ou non, susceptible de contenir des bactéries, par exemple une eau d'écoulement ou une eau de rinçage de fruits et légumes. Un tel matériau peut aussi être un mélange d'au moins deux matériaux tels que ci-dessus définis ; il peut donc être, notamment, soit préparé à partir de tissus, d'organes, de selles ou de liquides biologiques d'un malade atteint d'une affection, soit obtenu à partir de cultures "in vitro" ; un tel matériau biologique peut être aussi un sérum, du plasma, de l'urine, du liquide céphalo-rachidien, du liquide synovial, du liquide péritonéal, du liquide pleural, du liquide séminal ou du liquide ascétique.

On a déjà décrit une glycoprotéine plasmatique appelée β2-glycoprotéine I, ou encore en abrégé "β2GPI" ; la séquence de cette glycoprotéine humaine a été notamment indiquée dans les articles de J. LOZIER et coll., Proc. Natl. Acad. Sci. ISA, Vol. 81, p. 3640-3644 (juillet 1984), et de T. KRISTENSEN et coll., FEBS Letters" Vol. 289, p.183-186 (1991). Il a été constaté que cette protéine β2GPI présente un polymorphisme : la dénomination β2GPI sera considérée ci-après comme générique pour toutes les formes.

Dans la demande internationale WO 94/18569, on a indiqué que certains composés infectieux, en particulier protéiniques, se fixaient sur la forme de β2GPI qui avait été décrite dans le brevet français 2 701 263. On a proposé dans le document WO 94/18569, un procédé de détection et/ou de dosage de composés viraux dans lequel on fixe les composés infectieux viraux sur la forme de β2GPI utilisée ; on ajoute donc cette forme de β2GPI sur des composés infectieux viraux contenus dans un matériau biologique, de façon à séparer les composés viraux ainsi capturés pour ensuite les détecter et/ou les doser. Dans le brevet européen EP 775 315, on a décrit la formation d'un complexe entre un composé infectieux, en particulier protéinique, et une forme quelconque de β2GPI ; le composé infectieux pouvait, notamment, être une bactérie. Il ressort de ces documents que la β2GPI est susceptible de se fixer sur un support solide plan, tel que les fonds de puits d'une plaque de microtitration, et que la β2GPI ainsi accrochée sur ce support solide plan, est susceptible de fixer des bactéries présentes dans des échantillons cliniques, biologiques ou environnementaux à de très faibles concentrations. On sait, en outre, que de tels échantillons peuvent contenir des substances inhibant, au moins partiellement, la détection des pathogènes, substances qui, par conséquent, peuvent diminuer la sensibilité de la détection. Il est donc important de pouvoir capturer et concentrer ces pathogènes pour éliminer les substances qui inhibent leur mise en évidence.

Les études de la société demanderesse ont montré que la fixation de la β2GPI sur le fond des puits des plaques de titration, se faisait grâce à une conformation particulière de la β2GPI, conformation qui permettait ultérieurement la formation d'un complexe de la β2GPI avec un composé infectieux. La littérature avait d'ailleurs signalé que la conformation de la β2GPI variait à sa fixation sur une surface solide (Matsuura et autres, J. Exp. Med. 179, p. 457-462 (1994)). On avait déjà décrit (A. IWATA et autres, Biol. Pharm. Bull. 26(8), p. 1065-1069 (2003)) un procédé de concentration de virus utilisant des microbilles magnétiques sulfonées sur lesquelles les virus venaient s'accrocher, la concentration des virus étant obtenue grâce au fait que les microbilles étaient magnétiques et pouvaient être séparées du milieu infectieux par action d'un champ magnétique. Malheureusement le résultat de cette technique était essentiellement fonction de l'accrochage des virus sur les microbilles. Ce document explique de façon détaillée que certains virus non enveloppés ne se fixent pas sur des billes en polyéthylène-imine et qu'il est nécessaire d'utiliser des microbilles sulfonées pour concentrer certains virus. En outre, pour certains virus, il était nécessaire d'ajouter dans le milieu des cations bivalents. Il résulte de cette constatation que, selon la nature du virus, le polymère constituant les microbilles doit être différent, greffé ou non, et que des ions bivalents sont nécessaires ou non ; les billes doivent donc être préparées au coup par coup en fonction du virus à concentrer. Les mêmes constatations résultent du document E. UCHIDA et autres, Journal of Vïrological Methods, 143, p. 95-103 (2007), qui est relatif à la concentration des virus des hépatites A, B, C humaines. En présence d'un échantillon contenant un virus non identifié à détecter, il n'est pas possible de déterminer quelle nature de microbilles est susceptible de donner lieu à un accrochage du virus d'intérêt.

En conséquence, compte tenu des inconvénients existant pour la fixation de virus sur les microbilles, un homme de métier n'aurait pas été enclin à rechercher une fixation: de bactéries sur des microbilles. La société demanderesse a, néanmoins, été à l'encontre de ce préjugé défavorable en proposant, selon la présente invention, d'interposer, entre une microbille et une bactérie à fixer dessus, une molécule de β2GPI. L'état de la technique a permis de déterminer la nature des supports solides permettant un bon accrochage de la β2GPI ; la fixation de la β2GPI sur la microbille s'effectue alors sans que le polymère de la microbille ait à être modifié en fonction de la bactérie à fixer ultérieurement. Et, en outre, on a constaté que la fixation de la β2GPI sur la microbille ne perturbait pas l'accrochage de la bactérie sur la β2GPI ; or, ce dernier point était totalement inattendu car on ne pouvait pas prévoir que la conformation de la β2GPI fixée sur une microbille, permettrait l'accrochage d'un agent pathogène sur la glycoprotéine. Au demeurant et à titre complémentaire, un élément dissuasif pour aboutir à l'invention provenait du fait que l'on savait que la β2GPI avait une tendance à s'auto-polymériser (voir : Thrombosis Research,108, p. 175-180 (2003)), ce qui risquait d'entraîner une agglutination des microbilles porteuses de β2GPI, agglutination qui, bien entendu, rendait impensable la fixation d'agents pathogènes sur les molécules de β2GPI.

La présente invention a, en conséquence, pour objet un procédé de détection et/ou de quantification et/ou d'identification in vitro de bactéries présentes dans un milieu fluide M constituant un matériau biologique, procédé dans lequel, de façon connue, on prépare une suspension, dans un milieu liquide de suspension, de microbilles délimitées par une surface externe constituée d'un matériau polymère solide susceptible de fixer des protéines, caractérisé par le fait qu'il comporte les étapes suivantes :
a) dans un tampon approprié, on assure un chargement des microbilles de la suspension avec des protéines β2GPI par couplage avec une quantité suffisante de protéines β2GPI, soit de façon passive dans un milieu de suspension, soit en utilisant un protocole de liaison chimique connu ;
b) on met en contact, dans un conteneur, lesdites microbilles chargées en protéines β2GPI avec le milieu fluide M dans des conditions appropriées pour assurer, sans la présence d'ions de métal oxydant, une fixation suffisante des bactéries sur les protéines β2GPI portées par les microbilles ;
c) on sépare les microbilles ainsi préparées de leur milieu de suspension, on évacue ledit milieu de suspension hors du conteneur pour obtenir un résidu à forte concentration de bactéries ;
d) et on détecte et/ou quantifie et/ou identifie les bactéries du résidu.

Il a été tout à fait surprenant de constater que, lorsque l'on met en oeuvre le procédé ci-dessus défini et qu'on lave les microbilles constituant le résidu à forte concentration de bactéries, les bactéries captées par les microbilles conservent leur capacité de multiplication. Dans un mode avantageux de mise en oeuvre, le procédé selon l'invention est donc caractérisé par le fait qu'on lave les microbilles constituant le résidu, on les met en contact avec un milieu de culture susceptible de permettre leur multiplication et, de façon connue, on détecte et/ou quantifie et/ou identifie les bactéries à partir dudit milieu de culture. Pour permettre la multiplication des bactéries du milieu M, fixé sur les microbilles, on assure leur incubation, en aérobie ou anaérobie, sur le milieu de culture pendant un temps et à une température appropriés et on déduit des résultats de culture obtenus, l'existence et/ou la quantification et/ou l'identification desdites bactéries. Le milieu fluide M peut, notamment, être une hémoculture et, dans ce cas, après avoir obtenu le résidu à forte concentration de bactéries et l'avoir lavé, on peut remettre les microbilles en suspension dans un bouillon de culture, par exemple un bouillon tripticase-soja dit "BTS", et déposer ce bouillon sur un milieu de culture au sang, par exemple un milieu "Columbia" au sang de mouton.

On peut identifier les bactéries par coloration Gram et/ou par des subcultures, en milieu sélectif ou non ; on peut quantifier des bactéries par lecture de densité optique, par ATP-métrie ou par PCR.

On choisit, de préférence, le matériau solide constitutif de la surface externe des microbilles dans le groupe formé par les matières plastiques et les élastomères, ledit matériau portant ou non des groupements réactifs greffés sur la surface externe des microbilles pour assurer une liaison chimique avec les protéines β2GPI ; les microbilles peuvent avantageusement avoir une forme sensiblement sphérique et un diamètre moyen compris entre 1 et 100 000 nm. Selon une première variante, on sépare les microbilles de leur milieu de suspension par centrifugation ; mais selon une deuxième variante préférée, on choisit des microbilles ayant un coeur formé d'une (ou de) particule(s) de matériau magnétique pour permettre leur séparation par rapport au milieu de suspension grâce à un champ magnétique. De telles microbilles magnétiques sont disponibles dans le commerce : par exemple, elles sont constituées d'un coeur magnétique recouvert d'une matrice polymérique de polystyrène. Le champ magnétique permettant la séparation des microbilles par rapport à leur milieu de suspension, peut être créé par un simple aimant permanent que l'on approche du conteneur pour réaliser l'étape c) du procédé selon l'invention.

La seule limite,concernant le choix du matériau constitutif des microbilles, est de pouvoir coupler la β2GPI : on peut, par exemple, utiliser des microbilles magnétiques correspondant à la dénomination commerciale "Estapor® microsphères superparamagnétiques" vendues par la société "MERCK". Comme précédemment indiqué, le couplage de la β2GPI sur les microbilles peut se faire soit de façon passive, soit en utilisant un protocole de couplage chimique. Pour réaliser un couplage passif avec les microbilles "Estapor"® précitées, on met les microbilles en suspension dans un tampon contenant de la β2GPI, à un pH compris entre 3,5 et 10,5 et mieux, entre 5,5 et 9,5. Le tampon utilisé fait partie des tampons courants en biologie et, notamment, peut être un tampon acétate, phosphate, borate, Tris. Le mélange microbille/β2GPI est incubé à une température comprise entre 4°C et 40°C pendant un temps compris entre 10 mn et 24 h sous agitation, de préférence une agitation horizontale douce et constante. Par la suite, les microbilles sont séparées magnétiquement ou centrifugées et le surnageant est éliminé. Le culot contenant les microbilles est mis en suspension dans un tampon de conservation, qui est, de préférence, le même que celui utilisé ultérieurement pour le couplage, ce tampon ayant un pH compris entre 6 et 9. De préférence, on effectue le chargement des microbilles en protéines β2GPI en les mettant dans un milieu liquide de suspension qui contient, en solution aqueuse, de 10⁻⁶ à 100 mg de β2GPI par gramme de poids sec de microbilles, la concentration, en β2GPI du milieu étant alors comprise entre 10⁻⁵ et 10 µg/µl, et en agitant la suspension ainsi constituée pendant 15 à 60 mn à une température comprise entre 30°C et 45°C.

L'échantillon contenant le pathogène est mis en contact avec les microbilles chargées, soit directement, soit après sa dilution dans un tampon, dont le pH est compris entre 5 et 9, de préférence entre 5,6 et 8. Le complexe, qui se forme entre la β2GPI et le pathogène, est par la suite, incubé pendant une période de temps comprise entre 5 mn et 24 h, de préférence, entre 30 mn est 2 h, à une température comprise entre 4°C et 40°C, de préférence, environ 37°C. Après incubation, l'échantillon qui n'a pas réagi avec la β2GPI fixée sur les microbilles, est éliminé par centrifugation ou aimantation des microbilles. Les microbilles ainsi isolées peuvent être utilisées pour la détection et/ou la quantification du pathogène. La séparation et/ou le dosage et/ou la quantification du pathogène lié au support par la β2GPI peut se faire par tout moyen connu tel que l'infectiosité, une réaction enzymatique spécifique, un traceur fluorescent ou radiomarqué, la détection d'acide nucléique spécifique par hybridation avec une sonde marquée, une réaction en chaîne obtenue avec une polymérase (dite "PCR"), un dosage, une numération, une visualisation, un procédé optique, une microscopie électronique ou non.

Pour mieux faire comprendre l'objet de l'invention, on va en décrire maintenant, à titre d'exemples purement illustratifs et non limitatifs, plusieurs modes de mise en oeuvre.

### EXEMPLE 1 : Fixation d'une bactérie sur des microbilles chargées en β2GPI

La bactérie que l'on,a utilisée, est une souche d'Echerischia Coli (E. Coli) fournie par le Centre de conservation de produits agricoles (CPA). Une pré-culture est incubée à 37°C pendant 16 h dans du milieu LB (Luria Bertani) ayant la composition suivante :

| | |
|---|---|
| Bacto tryptone | 10 g |
| Extrait de levure | 5 g |
| NaCl | 10 g |
| pH | 7,5 |
| Eau | qsp 1 000 g |

Cette pré-culture est utilisée immédiatement ou conservée à 4,5°C.

Les microbilles destinées à fixer les bactéries que l'on utilise dans cet exemple sont des microbilles magnétiques vendues par la Société MERCK sous la dénomination "Estapor® microsphères superparamagnétiques" qui ont un diamètre compris entre 0,300 et 0,500 µm.

Ces microbilles sont mises en suspension dans un tampon acétate à un pH de 6,0 contenant la β2GPI. La concentration de β2GPI dans ce tampon de couplage est de 100 µg/ml ; les microbilles sont incubées dans le tampon sous agitation douce et constante à une température de 25°C pendant 3 heures. Les microbilles sont ensuite centrifugées à 1 500 tours/minute et le surnageant est éliminé ; le culot de centrifugation est mis en suspension dans le même tampon que celui utilisé pour le couplage de la β2GPI, ce qui forme la suspension de microbilles chargées en β2GPI que l'on veut tester.

Les cultures de bactéries à étudier sont placées dans des tubes à hémolyse de 1 ml avec des quantités de microbilles différentes suivant les tubes. Les tubes sont mis sous agitation horizontale pour bien mélanger les microbilles et chaque tube est incubé à 37°C ou à température ambiante (TA = 22°C) ; le temps d'incubation est variable suivant l'expérience réalisée. Dans chaque tube, on sépare ensuite les microbilles de la phase liquide au moyen d'un aimant placé extérieurement contre la paroi du tube et on mesure la densité optique (DO) du surnageant à 600 nm avec un spectrophotomètre "Eppendorf'.

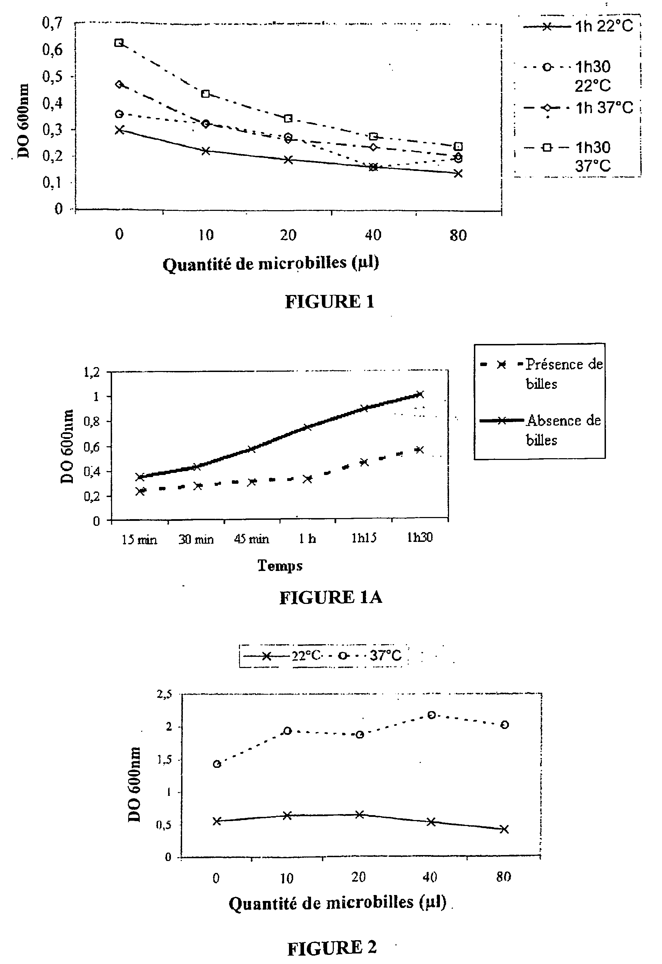

En l'absence de microbilles, la DO en début d'expérience, est égale à 0,2 et elle croît au cours du temps selon une croissance bactérienne normale ; en présence de microbilles, la DO reste presque stable pendant environ une heure puis elle croit comme en l'absence de microbilles (voir fig. 1A). Ceci suggère que les bactéries se sont fixées sur les microbilles, ce qui a retardé la croissance bactérienne normale. La figure 1 montre que, pour une même quantité de,microbilles et un même temps d'incubation, la DO est plus grande lorsque la température d'incubation est plus élevée, ce qui est normal pour une bactérie du tube digestif de mammifères, dont la croissance optimale se situe aux alentours de 37°C. On constate également, sur cette même figure, que pour une même température d'incubation, la DO est d'autant plus grande que le temps d'incubation est plus grand. On constate enfin sur la figure 1 que, pour une incubation de durée et de température données, la DO diminue lorsque la quantité de microbilles augmente.

On a également fait incuber les microbilles avec la culture tamponnée de E. Coli pendant 1 h 30 sous agitation. On sépare magnétiquement, comme précédemment, les microbilles et on écarte les surnageants ; on lave les microbilles avec du milieu de culture LB "frais" et avec une solution PBS correspondant à la formulation suivante :

| | |
|---|---|
| NaCl | 80 g |
| KCl | 74,562 g |
| KH₂PO₂ | 2,4 g |
| Na₂HPO₄/2H₂O | 29 g |
| Eau | qsp 1 000 g |

On laisse incuber le milieu de culture pendant une nuit à 37°C ou 20°C et on mesure la DO. Les résultats sont fournis sur la figure 2 en fonction de la quantité de microbilles introduite initialement dans la culture de E. Coli. On constate que la DO après incubation à 20°C évolue peu en fonction de la quantité de microbilles, alors qu'elle est croissante avec la quantité de microbilles pour une incubation à 37°C.

On a également étudié l'état physiologique des bactéries capturées par les microbilles. On effectue un dosage des ATP (adénosine tri-phosphate) et des nucléotides adényliques (NA) intracellulaires des bactéries. On sait que l'ATP est un indicateur spécifique de la cellule vivante car, après la mort cellulaire, il est très rapidement dégradé en ADP (adénosine di-phosphate) et AMP (adénosine monophosphate) par des ATPases. On sait, par ailleurs (demande de brevet français 04-11084 déposée le 19 octobre 2004) que la somme ATP + ADP + AMP reste constante et égale à NA au cours de la croissance cellulaire. On a mesuré par bioluminescence les quantités d'ATP et de NA présentes sur les microbilles après contact avec E. Coli.

Pour effectuer ces mesures, on utilise des tubes à hémolyse dans lesquels on met en place 10 µl de microbilles chargées de β2GPI que l'on incube avec 1 ml de la pré-culture bactérienne pendant différents temps d'incubation à une température de 37°C. Les billes sont ensuite séparées par aimantation (c'est-à-dire attraction par un aimant permanent extérieur au tube) pour récupérer le surnageant puis lavées avec du milieu frais. Après une nouvelle aimantation, on ajoute dans chaque tube 200 µl d'extractant et 1 ml d'une solution tampon, ces deux réactifs étant fournis par la société "Control Life Technologies". On laisse agir cet extractant pendant 10 mn pour :
- provoquer la rupture des enveloppes des bactéries afin de libérer les nucléotides ;
- inhiber rapidement les réactions enzymatiques, notamment ATPasiques ;
- avoir un effet destructeur minimal sur les NA.

On fractionne chaque échantillon en quatre parties de 100 µl que l'on met dans quatre petits tubes rhésus dont deux contiennent 5 µl de solution d'enzymes lyophilisés à savoir : phosphoénolpyruvate, adénylate kinase et pyruvate kinase ; dans les tubes à enzymes, on transforme l'AMP et l'ADP en ATP. On obtient donc deux tubes (SE) n'ayant pas subi l'action enzymatique et deux tubes (E) l'ayant subie. On ajoute aux quatre tubes 5 µl du détecteur lumineux d'ATP (luciférine/luciférase) et on passe un tube (E) et un tube (SE) dans un luminomètre (Control Life 300), où on mesure l'émission lumineuse pendant 5 s (résultat donné en unités relatives de lumière (URL)). Avec les deux tubes restants, on effectue une deuxième mesure après avoir ajouté 5 µl d'ATP standard (10 pmol/µl) dans chaque tube : on utilise le résultat pour corriger les erreurs dues à une inhibition éventuelle de l'émission lumineuse car la connaissance de la deuxième mesure permet de transformer la première en picomoles.

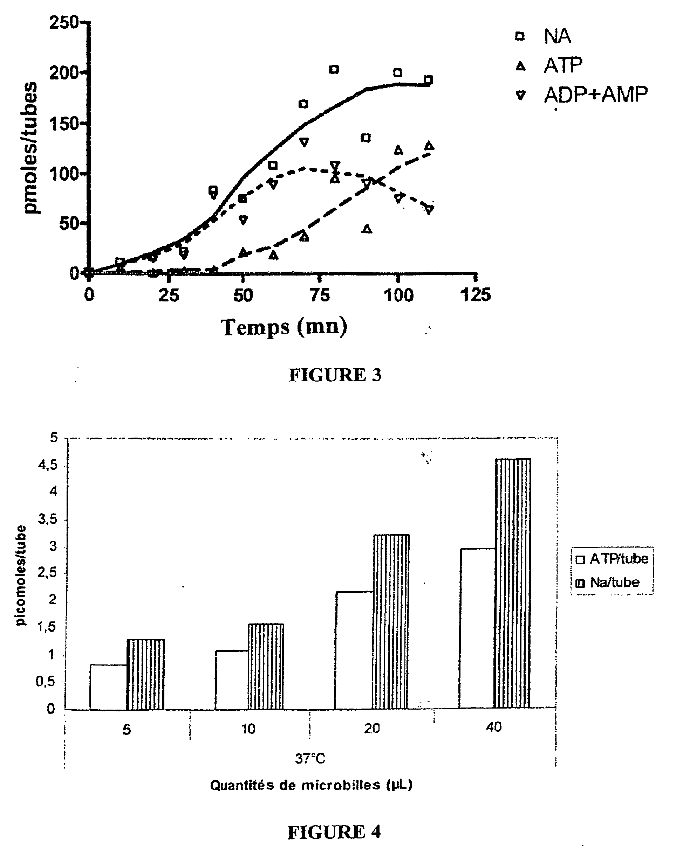

Les résultats sont donnés sur les figures 3 et 4.

Sur la figure 3, on voit une augmentation des NA intracellulaires présents sur les microbilles en fonction du temps et une saturation des microbilles en NA à partir d'un temps de contact de 80 mn environ entre les microbilles et la culture de bactéries initiale. La quantité d'ATP présente sur les microbilles augmente avec le temps de contact microbilles/culture ; on constate une saturation correspondant au fait que la capture des bactéries par les microbilles dépend de la surface des microbilles utilisées ; en outre, étant donné que la teneur en ATP d'une cellule est représentative de son activité, on peut aussi en déduire que les microbilles captent les bactéries et fixent les bactéries les plus actives.

Par ailleurs, les mesures d'ATP et de NA dans un tube, ont été faites en fonction de la quantité de microbilles utilisée dans le tube, l'incubation pour le captage des bactéries étant effectuée pour tous les tubes à 37°C pendant 1 h 30 sous agitation : les résultats sont fournis sur la figure 4. On constate une augmentation de l'ATP et des NA intracellulaires lorsque la quantité de microbilles augmente. Ceci confirme que les microbilles captent les bactéries présentes dans le milieu.

On sait que, pour les bactéries (voir D. CHAMPIAT, Biochimie luminescence et biotechnologie, Technoscope n° 51, Biofutur n° 110 et CHAMPIAT D. et LARPENT JP., Biochimie luminescence : Principes et applications, Edition Masson 1993), si la charge énergétique ATP/NA est comprise entre 0,5 et 0,75, les bactéries sont en phase de croissance. Le tableau I donné ci-après, a été établi en utilisant les valeurs expérimentales correspondant à la figure 4 et elles montrent que le rapport ATP/NA pour un contact microbilles/bactéries effectué à 37°C, est compris entre 0,5 et 0,7.

**TABLEAU I**

| | | | | |
|---|---|---|---|---|
| Température | 37°C | | | |
| Quantité de microbilles | 5 µl | 10 µl | 20 µl | 40 µl |
| ATP/NA | 0.64 | 0.69 | 0.67 | 0.64 |

Les microbilles fixent donc les bactéries en croissance, c'est-à-dire en pleine activité métabolique. Le captage de E. Coli sur les microbilles n'inhibe donc pas le métabolisme bactérien. Comme il a déjà été indiqué ci-dessus au sujet de la figure 2, les bactéries, qui ont été fixées sur les microbilles, génèrent, sur un milieu et à une température appropriés, une culture, dont la DO augmente en fonction de la concentration en microbilles, ce qui veut dire que, malgré leur captation par les microbilles, les bactéries continuent à se multiplier.

L'ensemble de ces résultats montre qu'il y a capture des bactéries par les microbilles jusqu'à une saturation due à la quantité de microbilles chargées en β2GPI que l'on met en oeuvre. Les microbilles n'inhibent pas la croissance bactérienne et n'entraînent pas une mortalité des bactéries captées.

Des essais analogues ont également été menés avec les bactéries *Pseudomonas aeruginosa, Streptococcus pneumoniae et Staphylococcus aureus* et ont donné le même type de résultats.

### EXEMPLE 2 : Interaction de la β2GPI avec les bactéries présentes dans le sang humain

Les microbilles utilisées sont les mêmes que celles dont la préparation a été détaillée dans l'exemple 1.

On a utilisé deux séries d'hémocultures (hémocultures I comprenant 5 échantillons et hémocultures II comprenant 35 échantillons) provenant de prélèvements hospitaliers. Ces hémocultures sont réalisées en mettant un prélèvement de sang veineux (environ 10 ml) dans des flacons aérobie et anaérobie de type BacT/ALERT®3D. Ces flacons sont ensuite incubés dans un automate à 35°C pendant au moins 5 jours. Les flacons sont équipés d'un système de détection colorimétrique grâce à un senseur situé à la base de chaque flacon. Le dioxyde de carbone produit par les bactéries en croissance, fait changer la couleur du senseur ; ce changement de couleur est détecté par l'automate et indique la présence d'une croissance bactérienne : ces hémocultures sont dites positives. Dans les flacons BacT/ALERT®3D se trouvent des particules de charbon actif, qui inhibent les antibiotiques potentiellement présents dans le sang des patients, la détection des microorganismes étant ainsi, améliorée. Pour confirmer la présence des bactéries dans les hémocultures qui ont été révélées positives dans l'automate, l'hôpital réalise une mise en culture sur gélose au sang. L'ensemble des résultats provenant des hôpitaux permet ainsi d'identifier
- des hémocultures positives (positives dans l'automate et positives en culture),
- des hémocultures négatives (négatives dans l'automate) et
- des hémocultures faussement positives (positives dans l'automate et négatives en culture).

Pour tester l'interaction des microbilles chargées de β2GPI avec les bactéries présentes dans le sang, on prélève 1 ml d'hémoculture pour chaque échantillon et on le met dans un tube de 15 ml. On ajoute différentes quantités de microbilles chargées en β2GPI et chaque tube est incubé à 37°C sous agitation horizontale. Les échantillons sont alors transvasés dans des tubes siliconés de 2 ml. Les tubes sont placés dans un champ magnétique qui retient les microbilles sur la paroi et on enlève le surnageant. Les microbilles sont ensuite lavées deux fois avec du PBS stérile de même composition que précédemment indiquée dans l'exemple 1 ; les microbilles sont ensuite remises en suspension dans 150 µl de milieu de culture BTS (Bouillon Tripticase-Soja) ayant la formulation suivante :

| | |
|---|---|
| Peptone de caséine | 17,0 g |
| Peptone de farine de soja | 3,0 g |
| D(+)-glucose | 2,5 g |
| Chlorure de sodium | 5,0 g |
| Phosphate dipotassique | 2,5 g |
| Eau | qsp 1 000 g |

Ce milieu de culture BTS a été porté à ébullition puis autoclavé pour le rendre stérile avant usage.

On prélève 50 µl de la suspension de microbilles ainsi obtenue et on les dépose dans une boîte de Petri sur un milieu "Columbia" au sang de mouton, dit « gélose au sang » (Laboratoires BioMérieux) ; cette gélose, de couleur rouge vif, contient des globules rouges : elle constitue un milieu riche, non sélectif, qui permet la croissance de la plupart des bactéries d'intérêt médical. Les boîtes de Pétri sont incubées à l'étuve à 37°C pendant 24 heures. Ce protocole permet avec les microbilles la détection des bactéries présentes dans les hémocultures. Trois méthodes de mises en évidence des bactéries capturées par les microbilles, ont été utilisées : ATP-métrie, mise en culture sur gélose au sang et PCR (Polymérase Chain Reaction) suivie ou non d'un séquençage.

### A) Hémocultures I

### a) ATP-métrie

La méthode d'ATP-métrie utilisée est identiquement la même que celle utilisée dans l'exemple 1. Pour chacun des échantillons 1,2,6,7, 8, on a prélevé 3 fois 1 ml que l'on a déposé dans des tubes de 15 ml, ce qui donne 3 sous-échantillons. On fait incuber les 15 sous-échantillons à 37°C, avec des temps d'incubation de 30, 60 ou 90 minutes pour les 3 sous-échantillons d'un même échantillon.

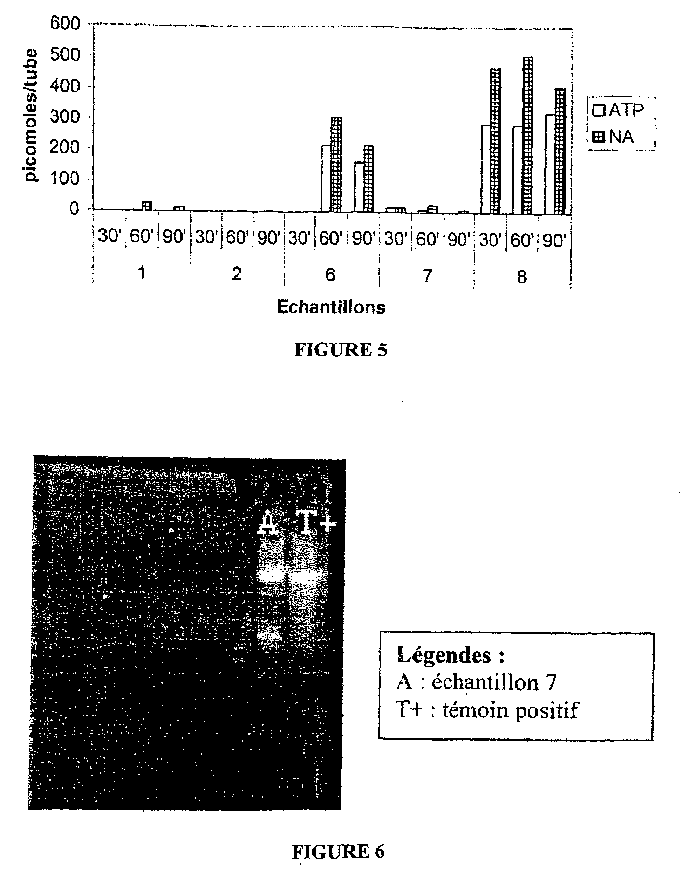

Aucune bactérie n'est détectée dans les échantillons 1, 2 et 7, indépendamment de la quantité de microbilles ou du temps d'incubation. En revanche, des bactéries sont détectées dans les échantillons 6 et 8 et les résultats sont fournis sur la figure 5. Ces résultats relatifs aux 5 échantillons correspondent à ceux obtenus par l'automate. Le tableau II ci-dessous donne les résultats correspondant aux calculs de charge énergétique afférents aux résultats de la figure 5 : on constate que les bactéries présentes dans les hémocultures 6 et 8 sont en phase de croissance.

**TABLEAU II**

| | Echantillon 6 | | | Echantillon 8 | | |
|---|---|---|---|---|---|---|
| Temps d'incubation sur sous-échantillon (en mn) | 30 | 60 | 90 | 30 | 60 | 90 |
| ATP/NA | / | 0.70 | 0.74 | 0.61 | 0.56 | 0.79 |

Cette première partie de l'exemple 2A) établit que les microbilles chargées en β2GPI captent les bactéries présentes dans les hémocultures.

### b) Mise en culture

On a mis en culture sur gélose au sang les bactéries fixées avec les différentes concentrations de microbilles dans les échantillons 1, 2, 6, 7, 8 comme indiqué sous a) ci-dessus. Les résultats obtenus sur ces échantillons après 24 h d'incubation à 37°C, sont présentés dans lé tableau III ci-après. Pour les hémocultures 6 et 8, la mise en culture confirme les résultats trouvés séparément à l'hôpital, ce qui démontre que les microbilles captent bien les bactéries présentes dans les tubes.

**TABLEAU III**

| N° d'échan tillon | Germes identifiés à l'hôpital | Condi tion de culture | Germes identifiés selon l'invention sur gélose au sang | | | | Coloration Gram |
|---|---|---|---|---|---|---|---|
| | | | Sans micro billes | Quantité de microbilles | | | |
| | | | | 25 µl | 50 µl | 75 µl | |
| 1 | négatif | aérobie | / | / | / | / | / |
| | | CO₂ | / | / | / | / | |
| 2 | négatif | aérobie | / | + | | / | Bacille (-)= pseudomonas |
| | | CO₂ | / | / | / | / | |
| 6 | Pseudo monas | aérobie | + | ++ | +++ | ++++ | Bacille (-)= pseudomonas |
| | | CO₂ | + | ++ | +++ | ++++ | |
| 7 | négatif | aérobie | / | / | 100 colonies avec hémolyses | 2/3 colonies avec hémolyses | Cocobacilles (-) |
| | | CO₂ | / | / | ++++ avec hémolyses | 4 colonies avec hémolyses | |
| 8 | S.mares cens | aérobie | + | ++ | +++ | ++++ | Bacille (-)= pseudomonas |
| | | CO₂ | + | ++ | +++ | ++++ | |

Pour identifier les colonies bactériennes, on a effectué des colorations de Gram et des sous-cultures sur différents milieux sélectifs ou non. Pour l'hémoculture n° 7, l'identification à l'hôpital donnait un résultat négatif alors que l'on obtient des colonies en utilisant des microbilles. On a effectué une coloration de Gram : le résultat a indiqué qu'il s'agissait d'un coccobacille Gram positif ; le coccobacille étant une forme intermédiaire entre un bacille et cocci, on l'a ensemencé sur les milieux suivants : milieu MacKonkey, milieu Chapman, milieu TS, milieu Cétrimide. Ces milieux correspondent aux formulations suivantes :

**TABLEAU IV**

| Milieu | Chapman | T.S. | MacConkey | Cétrimide |
|---|---|---|---|---|
| Peptone | 10 g | | 20 g | |
| Peptone trypsique de caséine | | 15 g | | |
| Peptone papaïnique de soja | | 5 g | | |
| Peptone de gélatine | | | | 16 g |
| Lactose | | | 10 g | |
| Sels biliaires n° 2 | | | 1,5 g | |
| Extrait de viande de boeuf | 1,0 g | | | |
| Chlorure de sodium | 75 g | 5 g | 5 g | |
| Mannitol | 10 g | | | |
| Rouge de phénol | 0,025 g | | | |
| Cristal violet | | | 0,001 g | |
| Rouge neutre | | | 0,05 g | |
| Bromure de tétradonium (cétrimide) | | | | 0,2 g |
| Acide nalidixique | | | | 15 g |
| Sulfate de potassium | | | | 10 g |
| Chorure de magnésium | | | | 1,4 g |
| Agar-agar | 15 g | | 15 g | |
| Agar (gélose) | | 15 g | | 10 g |

Les résultats ont montré que cette souche bactérienne poussait sur tous les milieux.

### c) Méthode par PCR et séquençage

On a ensuite effectué une PCR suivie d'un séquençage de l'ADNr 16S.

L'ADN bactérien est extrait à partir des bactéries qui ont été capturées par les microbilles ; les bactéries sont lysées en ajoutant aux microbilles 100 µl de "Chelex 30 %". Le mélange est incubé pendant 10 minutes à 95°C ; on effectue ensuite une centrifugation pendant 10 minutes à 10 000 tours/minutes. Le surnageant contenant l'ADN, est conservé à -20°C.

A 3 µl d'ADN extrait sont ajoutés 47 µl de la solution d'amplification (AquaPure Génomic DNA Isolation KIT) ; les concentrations finales sont les suivantes :
- 5 µl : dXTP 200 mM
- 10 µl: BUFFER 5X
- 5 µl: MgCl2 2mM
- 1 µl de chaque amorce : amorce diluée à 200 mL :
   27 f : GTGCTGCAGAGAGTTTGATCCTGGCTCAG
   1492 r : CACGGATCCTACGGGTACCTTGTTACGACTT
- 1 µl : Taq polymerase 5u/µL
- Eau PPI qsp 50µL

Après homogénéisation, les mélanges réactionnels sont placés dans un thermocycleur "Eppendorf" et soumis au programme suivant :

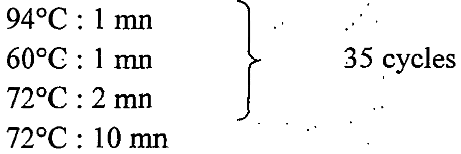

Les ADN sont ensuite maintenus à 10°C. La migration se fait sur un gel d'agarose à 2 % en tampon PBE 0,5X contenant du bromure d'éthydium. Le gel est alors observé sous lumière UV.

Les résultats de la PCR indiquent bien la présence d'une bactérie : on constate un fort signal positif (voir figure 6). L'identification de la bactérie peut se faire ensuite par séquençage de façon connue.

On constate donc que le procédé selon l'invention permet, grâce à l'utilisation de microbilles chargées en β2GPI, de détecter et identifier des bactéries dans le sang humain alors que les méthodes classiques mises en oeuvre à l'hôpital ne le permettent pas.

### B) Hémocultures II

On met en oeuvre la technique définie au début de l'exemple 2 : dans un tube de 15 ml, on dépose 1 ml d'une hémoculture et on y ajoute une certaine quantité de microbilles chargées en β2GPI (ici 25 ou 50 µl), qu'on laisse incuber dans le milieu. Puis on sépare magnétiquement les microbilles, on les lave et on les re-suspend dans un tampon stérile. On dépose cette suspension sur une gélose au sang dans une boîte de Pétri et on incube pendant 24 heures à 37°C. L'ensemble des hémocultures testées correspond au tableau V ci-après :

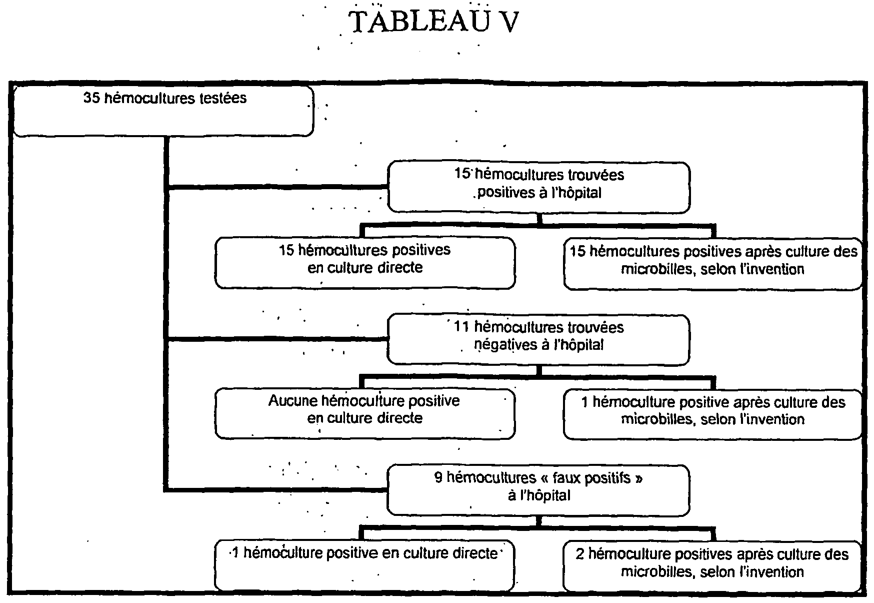

Pour les hémocultures positives, les microbilles permettent de confirmer les résultats obtenus par un automate et par culture à l'hôpital. Les microbilles captent donc bien les bactéries présentes dans les hémocultures.

Pour certains échantillons, avec 25 µl et 50 µl de microbilles, les résultats suggèrent la présence d'un deuxième type de bactéries non détecté à l'hôpital. Après identification, on a constaté qu'il s'agissait d'un staphylocoque (cocci à Gram positif). Pour les échantillons 5054 contenant *S. HOMINIS* et 5060 contenant *P. MIRABILIS* provenant de la même personne, on a mis en évidence sur gélose au sang les deux bactéries dans chacun des deux échantillons contrairement aux résultats donnés par l'hôpital.

Pour les hémocultures négatives, les billes ont permis de confirmer les résultats trouvés à l'hôpital, sauf pour une hémoculture 2081 où les microbilles mettaient en évidence des bactéries de type cocci à Gram positif (Staphylococus). Pour les faux positifs, les microbilles ont permis également de mettre en évidence des bactéries, de type cocci à Gram positif, pour deux des neuf hémocultures testées. On constate que parmi les hémocultures qui avaient été constatées négatives à l'hôpital, une hémoculture s'est révélée positive lorsque les microbilles sont cultivées sur gélose au sang, ce qui montre que l'invention permet d'améliorer la sensibilité de la détection.

Les tableaux VA, VB, VC et VD ci-après résument les résultats trouvés :

**TABLEAU VA**

| | | **Culture en condition aérobie :** | | | | | | | | | |
|---|---|---|---|---|---|---|---|---|---|---|---|
| | | **Germes identifiés sur gélose au sang, selon l'invention** | | | **Gram** | **Isolement de bactéries + Gram** | | | | | |
| **N° hémoc.** | **Germes identifiés à l'hôpital** | **Sans microbilles** | **25 µL de microbilles** | **50 µL de microbilles** | | **Sur gélose au sang** | | **Sur Chapman** | | **Sur M.K** | **Gram** |
| **1052** | **K.*pneumoniae*** | Colonie opaque : +++ | Colonie opaque : +++ | colonie opaque :+++ | | Colonie opaque | + + | | | | Bacille (-) = **K.*pneumoiae*** |
| | | colonie blanche: + | | colonie blanche:+ | | colonie blanche | + + | | | | Bacille (-) Bacille (-) = **K. *pneumoiae*** |
| **2058** | ***S.epidermidis*** | Colonie blanche : ++ Colonie jaune : + | Colonie blanche: ++ Colonie jaune : + | Colonie blanche : ++ Colonie jaune : + | Effectué dans la masse : Cocci (+) **S.*epidermidis*** et des billes | Colonie blanche | + | | | | |
| | | PS : présence d'hémolyse autour des colonies. | PS : présence d'hémolyse autour des colonies. | PS : présence d'hémolyse autour des colonies. | | Colonie jaune | + / | | | | Cocci (+) |
| **1481** | **E.*fecaelis*** | Colonie 1 : ++ | Colonie 1 : ++ | Colonie 1 : ++ | Colonie 1 : cocci (+) = E.fecaelis | Colonie 2 | + + | Colonie 2 | + | | Colonie 2 Cocci (+) = Staphylocoque |
| | | | | Colonie 2 : + | Colonie 2 cocci (+) | | | | | | |
| **7051** | **E.*coli*** | Colonie 1 : +++ | Colonie 1 : ++ | Colonie 1 : ++ | | | | | | | |

**TABLEAU VB**

| **Cultures en condition aérobie :** | | | | | | | | | | | | |
|---|---|---|---|---|---|---|---|---|---|---|---|---|
| | | **Germes identifiés sur gélose au sang, selon l'invention** | | | **Gram** | **Isolement de bactéries + Gram** | | | | | | |
| **N° hémo.** | **Germes identifiés à l'hôpital** | **Sans microbilles** | **25 µL de microbilles** | **50 µL de microbilles** | | **Sur gélose au sang** | | **Sur Chapman** | | **Sur M.K** | | **Gram** |
| **5054** | **S.*hominis*** | colonie 1 : +++ | colonie 1 : +++ | colonie 1 : +++ | Colonie 1 : Cocci (+) **S.*hominis*** | | | Colonie 1 | / | Colonie 1 | + | |
| | | | colonie 2 : + | colonie 2 : + | Colonie 2 : Bacille (-) **P.*mirabilis*** | | | Colonie 2 | ++ | Colonie 2 | / | |
| **4351** | **Faux +** | / | 25 colonies : certaines avec hémolyse et d'autres sans hémolyse | / | Coccii(+) | Avec hémo. | ++il y a hémolyse) | On isole une colonie | + | | | Cocci, (+) |
| | | | | | | Sans hemo. | ++ il y a hémolyse) | | | | | |
| **7059** | **E. *coli*** | Colonie 1 : ++ | Colonie 1 : + Colonie 2 : une seule colonie blanche | Colonie 1 ++ | Colonie 1 : coccobacille | Colon2 | + | Colonie 2 | ++ | Colonie 1 | 1 :++ | Colonie 2 : Cocci (+) Staphylocoque |
| | | | | | | | | | | | | Colonie 1 : bacille (-) **E. *coli*** |
| **50**60 | ***P.mirabilis*** | Colonie 1 : +++ | colonie 1 : +++ | colonie 1 : +++ | Colonie 2 : Cocci (+) **S.*hominis*** | | | Colonie 1 | + | Colonie 1 | / | |
| | | | colonie 2 : + | colonie 2 : + | Colonie 1: Bacille (-)= **P.*mirabilis*** | | | Colonie 2 | / | Colonie 2 | ++ | |

### Cultures en condition aérobie :

| TABLEAU VC | | | | | |
|---|---|---|---|---|---|
| | | **Germes identifiés sur gélose au sang, selon l'invention** | | | **Gram** |
| **N° hémo.** | **Germes identifiés à l'hôpital** | **Sans microbilles** | **25 µL de microbilles** | **50 µL de microbilles** | |
| **5055** | **S.*hominis*** | Colonie 1 : ++ | Colonie 1 : ++ | Colonie 1 : ++ | |
| **7096** | **S.*bovis*** | / | / | / | |
| **3353** | **E.*coli*** | Colonie 1 : ++ | Colonie 1 : ++ | Colonie 1 : ++ | |
| **3013** | **L.*monocytogenes*** | Colonie 1 : ++ | Colonie 1 : ++ | Colonie 1 : ++ | |
| **3053** | Corine ***bacterium.sp*** | après 24 h : / | après 24 h : / | après 24 h : / | Bacille (+) irrégulier qui pousse sur les microbilles. Donn donc l'impression d'une deuxième colonie |
| | | après 48 h : ++ | après 48h : ++ | après 48 h : ++ | |
| **2081** | **Négatif** | / | 8 colonies | 15 colonies | Cocci(+): staphylocoque |
| **2075** | **Négatif** | / | / | / | |
| **3060** | **Levures** | ++ | ++ | ++ | |
| **2080** | **Négatif** | / | / | / | |

### Cultures en condition anaérobie, aérobie et CO₂ :

| TABLEAU VD | | | | | |
|---|---|---|---|---|---|
| | | **Condition de culture** | **Germes identifiés sur gélose au sang, selon l'invention** | | |
| **N° hémo** | **Germes identifiés à l'hôpital** | | **Sans microbilles** | **25 µL de microbilles** | **50 µL de microbilles** |
| **1081** | **E.*coli*** | Aérobie | +++ | +++ | +++ |
| | | CO₂ | ++ | ++ | |
| | | Anaérobie | ++ | ++ | ++ |
| **6032** | **Enterobacter *cloaceae*** | Aérobie | +++ | +++ | +++ |
| | | Anaérobie | ++ | ++ | ++ |
| **4362** | **Pseudomas *aeruginosa*** | Aérobie | +++ | +++ | +++ |
| | | anaérobie | / | / | / |

## Revendications

1. Procédé de détection et/ou de quantification et/ou d'identification in vitro de bactéries présentes dans un milieu fluide M constituant un matériau biologique, procédé dans lequel, de façon connue, on prépare une suspension, dans un milieu liquide de suspension, de microbilles délimitées par une surface externe constituée d'un matériau polymère solide susceptible de fixer des protéines, **caractérisé par le fait qu'**il comporte les étapes suivantes :
a) dans un tampon approprié, on assure un chargement des microbilles de la suspension avec des protéines β2GPI par couplage avec une quantité suffisante de protéines β2GPI, soit de façon passive dans un milieu de suspension, soit en utilisant un protocole de liaison chimique connu ;
b) on met en contact, dans un conteneur, lesdites microbilles chargées en protéines β2GPI avec le milieu fluide M dans des conditions appropriées pour assurer, sans la présence d'ions de métal oxydant, une fixation suffisante des bactéries sur les protéines β2GPI portées par les microbilles ;
c) on sépare les microbilles ainsi préparées de leur milieu de suspension, on évacue ledit milieu de suspension hors du conteneur pour obtenir un résidu à forte concentration de bactéries
d) et on détecte et/ou quantifie et/ou identifie les bactéries du résidu.

2. Procédé selon la revendication 1, **caractérisé par le fait que** l'on lave les microbilles constituant ledit résidu, on les met en contact avec un milieu de culture susceptible de permettre leur multiplication et, de façon connue, on détecte et/ou quantifie et/ou identifie les bactéries à partir dudit milieu de culture.

3. Procédé selon la revendication 2, **caractérisé par le fait que** pour permettre la multiplication des bactéries du milieu M fixées sur les microbilles, on assure leur incubation sur le milieu de culture pendant un temps et à une température appropriés.

4. Procédé selon l'une des revendications 1 à 3, **caractérisé par le fait que** le milieu fluide M est une hémoculture.

5. Procédé selon la revendication 4, **caractérisé par le fait qu'**après avoir obtenu le résidu à forte concentration de bactéries, on remet les microbilles en suspension dans un bouillon Tripticase-Soja et on dépose ce bouillon sur un milieu de culture au sang de mouton.

6. Procédé selon l'une des revendications 1 à 5, **caractérisé par le fait que** l'on identifie les bactéries par coloration Gram et/ou par des subcultures sur milieu sélectif ou non.

7. Procédé selon l'une des revendications 1 à 6, **caractérisé par le fait que** l'on quantifie les bactéries par lecture de densité optique, par ATP-métrie ou par PCR de lysat.

8. Procédé selon l'une des revendications 1 à 7, **caractérisé par le fait que** l'on choisit le matériau solide constitutif de la surface externe des microbilles dans le groupe formé par les matières plastiques et les élastomères, ledit matériau portant ou non des groupements réactifs greffés sur la surface externe des microbilles pour assurer une liaison chimique avec les protéines β2GPI.

9. Procédé selon l'une des revendications 1 à 8, **caractérisé par le fait que** l'on choisit des microbilles ayant une forme sensiblement sphérique et un diamètre moyen compris entre 1 et 100 000 nm.

10. Procédé selon l'une des revendications 1 à 9, **caractérisé par le fait que** l'on choisit des microbilles ayant un coeur formé d'une (ou de) particule(s) de matériau magnétique pour permettre leur séparation par rapport au milieu de suspension grâce à un champ magnétique.

11. Procédé selon l'une des revendications 1 à 9, **caractérisé par le fait que** l'on sépare les microbilles de leur milieu de suspension par centrifugation.

12. Procédé selon l'une des revendications 1 à 11, **caractérisé par le fait que** l'on effectue le chargement des microbilles en protéines β2GPI en les mettant dans un milieu liquide de suspension qui contient, en solution aqueuse, de 10⁻⁶ à 100 mg de β2GPI par gramme de poids sec de microbilles, la concentration du milieu en β2GPI étant comprise entre 10⁻⁵ et 10 µg/µl, et en agitant la suspension ainsi constituée pendant 15 à 60 minutes à une température comprise entre 30 et 45°C.

## Claims

1. *In vitro* method for detection and/or quantification and/or identification of bacteria present in a fluid medium M constituting a biological material, a method in which, in a known manner, a suspension of microbeads in a liquid suspension medium is prepared, said microbeads being delimited by an outer surface constituted by a solid polymer material capable of binding proteins, **characterized by** the fact that it comprises the following steps:
a) in an appropriate buffer, a loading of the microbeads in the suspension with β2GPI proteins is ensured by coupling with a sufficient quantity of β2GPI proteins, either passively in a suspension medium or using a known chemical binding protocol;
b) in a container, said microbeads loaded with β2GPI proteins are brought into contact with the fluid medium M under appropriate conditions in order to ensure, without the presence of oxidizing metal ions, sufficient binding of the bacteria to the β2GPI proteins carried by the microbeads;
c) the microbeads thus prepared are separated from their suspension medium, said suspension medium is removed from the container in order to obtain a residue with a high concentration of bacteria;
d) and the bacteria of the residue are detected and/or quantified and/or identified.

2. Method according to claim 1, **characterized by** the fact that the microbeads constituting said residue are washed, brought into contact with a culture medium capable of allowing their multiplication and, in a known manner, the bacteria are detected and/or quantified and/or identified from said culture medium.

3. Method according to claim 2, **characterized by** the fact that in order to allow the multiplication of the bacteria of the medium M bound to the microbeads, their incubation on the culture medium is ensured for an appropriate period and at an appropriate temperature.

4. Method according to one of claims 1 to 3, **characterized by** the fact that the fluid medium M is a haemoculture.

5. Method according to claim 4, **characterized by** the fact that after obtaining the residue with a high concentration of bacteria, the microbeads are resuspended in a trypticase soy broth, and this broth is applied to a sheep blood culture medium.

6. Method according to one of claims 1 to 5, **characterized by** the fact that the bacteria are identified by Gram staining and/or by sub-cultures on selective or non-selective medium.

7. Method according to one of claims 1 to 6, **characterized by** the fact that the bacteria are quantified by optical density reading, by ATP-metry or by PCR of lysate.

8. Method according to one of claims 1 to 7, **characterized by** the fact that the solid material constituting the outer surface of the microbeads is chosen from the group formed by the plastics and the elastomers, said material carrying or not carrying reactive groups grafted to the outer surface of the microbeads in order to ensure a chemical bond to the β2GPI proteins.

9. Method according to one of claims 1 to 8, **characterized by** the fact that microbeads having a substantially spherical shape and an average diameter comprised between 1 and 100,000 nm are chosen.

10. Method according to one of claims 1 to 9, **characterized by** the fact that microbeads having a core formed by one (or more) particle(s) of magnetic material are chosen in order to allow their separation from the suspension medium using a magnetic field.

11. Method according to one of claims 1 to 9, **characterized by** the fact that the microbeads are separated from their suspension medium by centrifugation.

12. Method according to one of claims 1 to 11, **characterized by** the fact that the loading of the microbeads with β2GPI proteins is carried out by placing them in a liquid suspension medium which contains, in aqueous solution, from 10⁻⁶ to 100 mg of β2GPI per gram of dry weight of microbeads, the concentration of β2GPI in the medium being comprised between 10⁻⁵ and 10 µg/µl and by stirring the suspension thus constituted for 15 to 60 minutes at a temperature comprised between 30 and 45°C.

## Patentansprüche

1. Verfahren zum Nachweisen und/oder Quantifizieren und/oder Identifizieren, in vitro, von Bakterien in einem ein biologisches Material bildenden flüssigen Medium M, wobei bei dem Verfahren auf bekannte Weise in einem flüssigen Suspensionsmedium eine Suspension von Trägerkügelchen hergestellt wird, die von einer Außenfläche begrenzt werden, die von einem festen Polymermaterial gebildet wird, das Proteine fixieren kann, **dadurch gekennzeichnet, dass** es die folgenden Schritte beinhaltet:
a) Beladen, in einem geeigneten Puffer, der Trägerkügelchen der Suspension mit β2GPI-Proteinen durch Kopplung mit einer ausreichenden Menge an β2GPI-Proteinen, entweder auf passive Weise in einem Suspensionsmedium oder mittels eines bekannten chemischen Verbindungsschemas;
b) Inkontaktbringen, in einem Behälter, der genannten mit β2GPI-Proteinen beladenen Trägerkügelchen mit dem flüssigen Medium M unter geeigneten Bedingungen, um in Anwesenheit von Ionen von oxidierendem Metall eine ausreichende Fixierung von Bakterien an den von den Trägerkügelchen getragenen β2GPI-Proteinen zu erzielen;
c) Trennen der so hergestellten Trägerkügelchen von ihrem Suspensionsmedium, Evakuieren des genannten Suspensionsmediums aus dem Behälter, um einen Rest mit hoher Bakterienkonzentration zu erhalten; und
d) Nachweisen und/oder Quantifizieren und/oder Identifizieren der Bakterien des Rests.

2. Verfahren nach Anspruch 1, **dadurch gekennzeichnet, dass** die den genannten Rest bildenden Trägerkügelchen gewaschen, mit einem Kulturmedium für deren Vermehrung in Kontakt gebracht und die Bakterien von dem genannten Kulturmedium auf bekannte Weise nachgewiesen und/oder quantifiziert und/oder identifiziert werden.

3. Verfahren nach Anspruch 2, **dadurch gekennzeichnet, dass** zum Vermehrenlassen der an den Trägerkügelchen fixierten Bakterien des Mediums M deren Inkubation auf dem Kulturmedium für eine geeignete Zeit bei einer geeigneten Temperatur bewirkt wird.

4. Verfahren nach einem der Ansprüche 1 bis 3, **dadurch gekennzeichnet, dass** das flüssige Medium M eine Hämokultur ist.

5. Verfahren nach Anspruch 4, **dadurch gekennzeichnet, dass** die Trägerkügelchen nach dem Erhalten des Rests mit hoher Bakterienkonzentration in Suspension in eine Trypticase-Soja-Brühe gebracht werden und diese Brühe auf ein Kulturmedium mit Schafsblut aufgebracht wird.

6. Verfahren nach einem der Ansprüche 1 bis 5, **dadurch gekennzeichnet, dass** die Bakterien durch Gram-Färbung und/oder durch Subkulturen auf selektivem oder nicht selektivem Medium identifiziert werden.

7. Verfahren nach einem der Ansprüche 1 bis 6, **dadurch gekennzeichnet, dass** die Bakterien durch Lesen der optischen Dichte, durch ATP-Metrik oder durch Lysat-PCR quantifiziert werden.

8. Verfahren nach einem der Ansprüche 1 bis 7, **dadurch gekennzeichnet, dass** das die Außenfläche der Trägerkügelchen bildende feste Material ausgewählt wird aus der Gruppe bestehend aus Kunststoffen und Elastomeren, wobei das genannte Material reaktionsfähige Gruppen trägt oder nicht, die auf die Außenfläche der Trägerkügelchen aufgepropft sind, um eine chemische Verbindung mit den β2GPI-Proteinen zu erzielen.

9. Verfahren nach einem der Ansprüche 1 bis 8, **dadurch gekennzeichnet, dass** Trägerkügelchen mit einer im Wesentlichen sphärischen Form und einem mittleren Durchmesser zwischen 1 und 100.000 nm gewählt werden.

10. Verfahren nach einem der Ansprüche 1 bis 9, **dadurch gekennzeichnet, dass** Trägerkügelchen mit einem Kern gewählt werden, der aus einem oder mehreren Partikeln aus magnetischem Material gebildet ist, um deren Trennung in Bezug auf das Suspensionsmedium mittels eines magnetischen Feldes zuzulassen.

11. Verfahren nach einem der Ansprüche 1 bis 9, **dadurch gekennzeichnet, dass** die Trägerkügelchen durch Zentrifugieren von ihrem Suspensionsmedium getrennt werden.

12. Verfahren nach einem der Ansprüche 1 bis 11, **dadurch gekennzeichnet, dass** das Beladen der Trägerkügelchen mit β2GPI-Proteinen dadurch bewirkt wird, dass sie in ein flüssiges Suspensionsmedium gegeben werden, das in wässriger Lösung 10⁻⁶ bis 100 mg β2GPI pro Gramm Trockengewicht Trägerkügelchen enthält, wobei die Konzentration des β2GPI-Mediums zwischen 10⁻⁵ und 10 µg/µl liegt, und die so gebildete Suspension 15 bis 60 Minuten lang bei einer Temperatur zwischen 30 und 45°C gerührt wird.
